(19) Europäisches Patentamt
European Patent Office
Office européen des brevets

(11) Publication number: **0 050 952**
**B1**

(12) **EUROPEAN PATENT SPECIFICATION**

(45) Date of publication of patent specification: **21.09.83**

(51) Int. Cl.³: **C 07 H 5/02, A 23 L 1/236**

(21) Application number: **81304876.6**

(22) Date of filing: **19.10.81**

(54) **Sweet chlorine-substituted disaccharides.**

(30) Priority: **28.10.80 GB 8034666**
**21.08.81 GB 8125621**

(43) Date of publication of application:
**05.05.82 Bulletin 82/18**

(45) Publication of the grant of the patent:
**21.09.83 Bulletin 83/38**

(84) Designated Contracting States:
**AT BE CH DE FR IT LI LU NL SE**

(56) References cited:
**FR - A - 2 337 762**

(73) Proprietor: **TATE & LYLE PUBLIC LIMITED COMPANY**
**Sugar Quay Lower Thames Street**
**London, EC3R 6DQ (GB)**

(72) Inventor: **Lee, Cheang Kuan**
**92, Linden Drive**
**Singapore 1128 (GB)**

(74) Representative: **Ablewhite, Alan James et al,**
**MARKS & CLERK 57/60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 050 952 B1

Courier Press, Leamington Spa, England

# Sweet chlorine-substituted disaccharides

This invention relates to new compounds derived from sucrose which are high potency sweeteners. The invention also relates to compositions containing the sweeteners and to sweetening methods using them.

Although sucrose is still the most widely used sweetening agent, many efforts have been made to find substantially sweeter alternatives which could be used when it is desired to combine a high degree of sweetness with a low calorie content and/or a low risk of dental caries, e.g. in dietetic products and in the manufacture of soft drinks. The two most commerically successful non-sucrose sweeteners (that is to say sweeteners comprising a compound other than sucrose itself) to date have been saccharin and cyclamate, having respectively about 200 and about 30 times the sweetening power of sucrose. However, the use of these sweeteners, particularly cyclamate, has recently been restricted or banned in some countries because of doubts about their safety. Saccharin also suffers from the disadvantage of an unpleasantly bitter after-taste which can be detected by many people.

More recently, many other non-sucrose sweeteners have been investigated, some of natural origin and others synthetic, covering a wide range of chemical structures. These compounds have included proteins such as monellin, thaumatin and miraculin; dipeptides such as aspartame, and dihydrochalcones such as neohesperidine dihydrochalcone. However, apart from the difficulties of synthesising or extracting such sweeteners, there is the problem that they do not necessarily possess the same quality of sweetness as sucrose. In particular, as compared with sucrose, the sweetness may be slow in onset and relatively lingering, and there may be a liquorice-like or other after-taste, making the sweeteners unsuitable as a direct replacement for sucrose unless these differences can be masked.

Although numerous sweeteners of widely diverse chemical structures have now been investigated, it is significant to note that sweetness substantially greater than that of sucrose has been discovered in only one very small group of derivatives of sucrose and in no other carbohydrate. Those intensely sweet substances that are known are generally not carbohydrates at all. Indeed, the presence of some substituents on the sucrose molecule is known to destroy its sweetness. Thus, for example, esterification in various positions yields products which are non-sweet. The loss of sweetness in sucrose derivatives in which hydroxy groups were replaced by other functions comfirmed the theory of Shallenberger (J. Food Sci. (1963) *28*, 584) that sweetness of sugars was caused by hydrogen bonding of suitably placed hyroxy groups with the receptor site. Research in support of this theory was carried out by various workers by removing the hydroxy groups from sucrose and by altering their configuration. In every case, removal of hydroxy groups either reduced sweetness or left it substantially unaltered.

Then, towards the end of 1975, some very sweet sucrose derivatives were discovered. British Patent 1 543 167 discloses and claims a particular class of chlorinated derivatives of sucrose which were found to possess a high degree of sweetness, in some instances several hundred times that of sucrose, and yet to have the same quality of sweetness as sucrose with the absence of any delay in onset or unpleasant after-taste. The compounds in patent 1 543 167 have the hydroxy groups at certain combinations of the 4-, 6-, 1′-, and 6′- positions replaced by chlorine atoms. The positioning of the chloro substituents was found to be critical in that only certain combinations of chloro substituents gave sucrose derivatives with a high degree of sweetness: other combinations removed the sweetness of sucrose as would have been expected.

In the years following 1975, no further very sweet derivatives of sucrose have been discovered. Indeed, the only further chlorinated derivatives of sucrose assessed in that time are the 2,1′dichloro-2,1′-dideoxy derivative, which is not very sweet and the 2,6,1′,6′-tetrachloro-2, 6,1′,6′-tetradeoxy derivatives disclosed in U.K. Patent application 2037561A. In complete contrast to the compounds of patent 1 543 167, this 2,6,1′,6′-tetrachloro- derivative of sucrose was found to be a potent bittering agent, having a bittering power comparable with that of quinine. It thus appeared that departure from the chlorine substitution of the patent 1 543 167 would lead to compounds which are not sweet but which might instead possess other organoleptic properties.

Very surprisingly, we have now found some chlorosucrose derivatives possessing a different pattern of chlorine-substitution but neverthless possessing an intense sweetness.

According to the present invention we provide a 4-chloro-4-deoxy-α-D-galactopyranosyl 1,4,6-trichloro-1,4,6-trideoxy-β-D-hexulofuranoside of the general formula

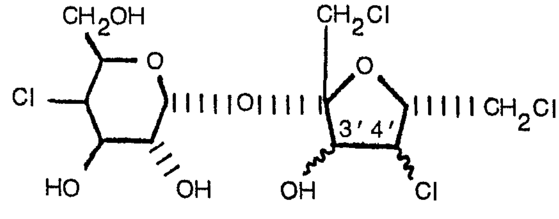

I

(in which the 3′ and 4′- substituents have a trans configuration relative to each other) especially 1,4,6-trichloro-1,4,6-trideoxy-β-D-fructofuranosyl 4-chloro-4-deoxy-α-D-galactopyranoside, but also 4-chloro-4-deoxy-α-D-galactopyranosyl 1,4,6-trichloro-1,4,5-trideoxy-β-D-sorbofuranoside.

These novel compounds have been found in informal taste tests to possess a degree of sweetness of similar order to 1,6-dichloro-1,6-dideoxy-β-D-fructofuranosyl 4-chloro-4-deoxy-α-D-galactopyranoside, otherwise known as 4,1′,6′-trichloro-4,1′,6′-trideoxy-*galacto*sucrose, also referred to as TGS; i.e. a sweetness of the order of several hundred times that of sucrose when compared at threshold levels or as, say, 5% solutions. In particular, 1,4,6-trichloro-1,4,6-trideoxy-β-D-fructofuranosyl 4-chloro-4-deoxy-α-D-galactopyranoside has been found to possess a sweetness about 220 times that of sucrose when compared at a concentration of 6.7% Thus, for example, in a typical test, a 0.003% solution of the compound is compared by a panel of tasters with sucrose solutions at 5, 6, 7, 8 and 9% concentrations. The average sucrose concentration which matches the test solution is then divided by 0.003 to give the approximate number of times the test compound is sweeter than sucrose. The *sorbo* isomer has a sweetness of about 200 times that of sucrose on a similar basis.

The new compounds according to the present invention can be obtained by direct chlorination of a sucrose derivative blocked in the 6-position, e.g. by esterification, but free in the 4′-position and in the 4-, 1′- and 6′-positions, and subsequent removal of the blocking group from the 6-position. A preferred chlorination reaction of this type uses sulphuryl chloride in the presence of an organic base such as pyridine and a suitable solvent, e.g. a chlorinated hydrocarbon such as chloroform. Some by-products, such as TGS, will also be formed in this reaction, and it is believed that the chlorination probably proceeds at the 4-, 1′- and 6′-positions more rapidly than at the 4′-position. It is also believed that the chlorination at the 4′-position involves a different mechanism from that at the other positions and proceeds *via* the formation of a 3′, 4′-epoxide. Alternatively a sucrose derivative already carrying some chlorine atoms can be used as starting material, e.g. a 6- ester of TGS.

Preferably, the reaction is arranged so that the sulphur trioxide evolved from the reaction mixture is led out of the reaction vessel via a drying tube so as to prevent formation of sulphuric acid which might run back into the reaction mixture, e.g. with refluxing solvent. Typically, 8 molar equivalents of sulphuryl chloride are allowed to react with sucrose in pyridine/chloroform at about 40—50°C for several hours. The reaction mixture may be worked up as is usual for a sulphuryl chloride reaction, e.g. by quenching with methanol, dechlorosulphation with a trace of sodium iodide and acetylation followed by separation by chromatography, crystallisation etc.

The product obtained from this process when applied to sucrose 6-acetate has, in fact, been found to comprise 2 closely related isomers and it is believed that these are two configurational isomers on the 3′ and 4′ positions. While we do not wish to be bound by theory, it is believed that the epoxide formation can yield either the *lyxo*- or *ribo*-epoxides which in turn, on ring opening, yield the 4′-chloro-4′-deoxy-fructose and 4′-chloro-4′deoxy-sorbose derivatives.

An alternative method of preparation, especially for the fructoisomer comprises the chlorination of a 4′-chloro-4′-deoxysucrose derivative esterified or otherwise blocked in the 6-position and having free hydroxy groups in the 4-, 1′ and 6′-positions. Any convenient chlorinating agent may be used, for example sulphuryl chloride, or a Vilsmeier reagent, e.g. an N,N-dialkyl-(chloromethaniminium) chloride obtained by the reaction of an inorganic acid chloride with an N,N-dialkylformamide or N,N-dialkylacetamide. 4′-Chloro-4′-deoxysucrose (i.e. 4-chloro-4-deoxy-β-D-fructofuranosyl-D-glucoyranoside) itself is a known compound (Guthrie *et al.* Carbohydrate Research 75(1979) pp C1 to C4). The compound obtained by this alternative method is that in which the 3′- and 4′- substituents are in the fructose configuration.

However, yet another method, which is selective for the preparation of the 4′-chloro-4′-deoxy*fructo* derivative, is namely a process for the preparation of 1,4,6-trichloro-1,4,6-trideoxy-β-D-fructofuranosyl 4-chloro-4-deoxy-α-D-galactopyranoside (i.e. 4,1′,4′,6′-tetrachloro-4,1′,4′,6′-tetradeoxy*galacto*-sucrose), in which the 4′-chloro substituent is introduced into the fructose ring of a sucrose derivative without inversion of configuration *via* formation of a 3′,4′-*lyxo*epoxide, by reaction of a compound of the general formula

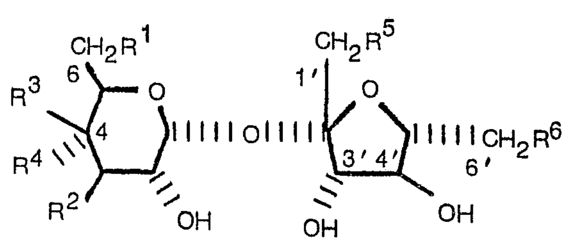

II

(in which $R^1$ and $R^2$ each represent a hydroxy group or a protected hydroxy group, $R^3$ represents a chlorine atom and $R^4$ a hydrogen atom, or $R^3$ represents a hydrogen atom and $R^4$ a hydroxy group; and $R^5$ and $R^6$ each represent a hydroxy group or a chlorine atom) with a *lyxo*-epoxide-forming reagent such as

triphenyl phosphine (TPP) in combination with a potentiator such as diethylazodicarboxylate (DEAD), protection of all reactive hydroxy groups in the molecule, e.g. by esterification, reaction of the epoxide with a source of chloride ions in a polar aprotic solvent such as DMF, and removal of protecting groups, hydroxy groups in any of the 4, 1′ and 6′-positions being subsequently replaced by chlorine atoms by a method known *per se.*

The process is conveniently effected using as the starting material a sucrose derivative in which chloro substituents are present at those other positions required. Free hydroxy groups at the 3- and 6-positions lead to the formation of unwanted 3,6-anhydro- by-products and for this reason it is much preferred to protect one or both of these positions, e.g. by esterification or etherification, and of the two, the 6-position is easier to protect. Thus, one particularly preferred group of starting materials are those compounds of the general formula

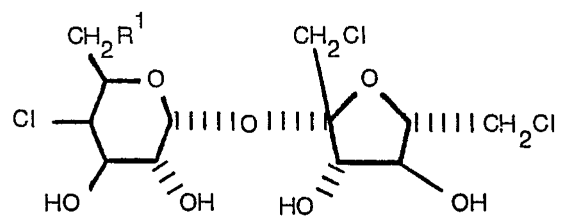

IIa

(in which $R^1$ represents a protected hydroxy group). One particularly useful class of protected hydroxy groups are the aryl and/or alkyl-substituted silyloxygroups, for example the *t*-butyl diphenyl silyloxy group. Other protected hydroxy groups include acyloxy groups, especially aliphatic or aromatic carboxylic acyloxy groups such as benzoyloxy groups or acetoxy groups.

The *lyxo*-epoxide forming reaction is conveniently effected in an inert solvent, e.g. a hydrocarbon such as toluene, depending on the solubility of the starting material. The DEAD is preferably brought into contact with the starting material in the solvent and the TPP is then added to the mixture. The reaction is exothermic and the mixture may be maintained hot (e.g. at reflux) for a suitable period of, say, 1 to 5 hours. The reaction mixture may then be worked up by quenching with an alkanol, e.g. methanol, and separating the components, e.g. by chromatography.

The protection of the hydroxy groups before reaction with the source of chloride ions is conveniently effected by acylation, especially acetylation by reaction with acetic anhydride. It is also convenient that the protection at position 6 during this reaction should be by acetylation. Thus, if the 6-position has previously been protected with a silyl group, it is desirable to remove this and acylate instead.

The compounds according to the invention are non-toxic, having an $LD_{50}$ (acute oral) in mice of over 1g/kg.

According to a further feature of the present invention there are provided ingestible products and oral compositions containing at least one of the above mentioned novel compounds as a sweetening agent. By the term "ingestible product" there is meant one which in the ordinary course of use is intended to be swallowed, for instance a foodstuff or beverage, or an orally administered pharmaceutical composition. By "an oral composition" there is meant one which in the ordinary course of use is not intended to be ingested as such, but is taken into the mouth for the treatment of the throat or buccal cavity, for instance a toothpaste, tooth powder, mouthwash, gargle, troche, dental lotion or chewing gum.

According to the present invention there is also provided a sweetening composition comprising at least one of the above mentioned novel compounds together with a solid extender or carrier, or a liquid extender or carrier. By a "sweetening composition" there is meant a composition which is not itself taken orally, to be by ingested or held in the mouth, but instead is intended to be added to other ingestible products or oral compositions to render them sweet, or to increase their sweetness.

The extender or carrier referred to above comprises any suitable vehicle for the sweet compound so that it can be formulated in a composition which can conveniently be used for sweetening other products, e.g. granules, tablets or a solution in a dropper pack. The extender or carrier may thus include, e.g., conventional water-dispersible tabletting ingredients, such as starch, lactose and sucrose itself, low density bulking agents to provide a granular sweetening composition having a volume per unit sweetness equivalent to that of sucrose, e.g., spray dried maltodextrins, and aqueous solutions containing adjuvants such as stabilizing agents, colouring agents and viscosity-adjusting agents.

Beverages, such as soft drinks, containing the above-mentioned sweet compound may be formulated either as sugar-free dietetic products, or "sugar-reduced" products containing the minimum amount of sugar required by law. In the absence of sugar it is desirable to add further agents to provide a "mouthfeel" similar to that provided by sugar, e.g. pectin or a vegetable gum. Thus, pectin may be added at a level of 0.1 to 0.15% in a bottling syrup.

According to a further feature of the present invention there is provided a method of sweetening a substance comprising incorporating therein a novel compound mentioned above.

The following examples illustrate the invention further (temperatures are given in degrees centigrade):—

Example 1

1,4,6-Trichloro-1,4,6-trideoxy-β-D-fructofuranosyl 4-chloro-4-deoxy-α-D-galactopyranoside and the corresponding *sorbo* isomer

Sucrose 6-acetate (75g) was dissolved in a mixture of pyridine (187.5ml) and chloroform (187.5ml) and the solution cooled to −75°. Sulphuryl chloride (221ml, approximately 14 M.E.) was added dropwise with stirring and then the reaction mixture was slowly allowed to warm to room temperature. The reaction vessel was fitted with a guard tube of calcium chloride to prevent intake of water vapour. The reaction mixture was heated 45° for about 24 hours. The mixture wa then poured into a 10% mixture of sulphuric acid and ice (51) with vigorous stirring. The product was extracted into dichloromethane (3 x 1 l) which was washed with water, sodium bicarbonate, water and dried over sodium sulphate. The organic extract was concentrated to dryness, dissolved in methanol (1 l) and a few crystals of sodium iodide were added to ensure complete de-chlorosulphation. After being left for 30 min, the solution was concentrated to dryness, dissolved in pyridine (1 l) and the dissolved material was acetylated by addition of acetic anhydride (200 ml). The reaction was stirred at room temperature for 16h then poured into ice/water (51). The precipitated product was filtered off dissolved in dichloromethane, dried over sodium sulphate, and concentrated to a syrup which was eluted from a silica gel column using 60—80 petroleum ether: ethyl acetate (2:1). An incomplete separation was observed and a second column was run using 60—80 petroleum ether: ethyl acetate (3:1). The major component isolated was still a mixture of two compounds with very similar polarities.

This mixture was left to crystallise from diethyl ether and it was observed that two different types of crystals formed. After all the solvent had evaporated, the two crystalline types were separated by hand. Tlc (diethyl ether—petrol 4:1) revealed little or no cross contamination. The slightly faster moving major component was show to be 4,1′,4′,6′-tetrachloro-4,1′,4′,6′-tetradeoxy*galactosorbo*sucrose tetra-acetate and the slower moving minor component was shown to be 4, 1′,4′,6′-tetrachloro 4,1′,4′,6′-tetradeoxy*galacto*sucrose tetra-acetate.

De-esterification was then achieved as follows:

A solution of 4,1′,4′,6′-tetrachloro- 4,1′,4′,6′-tetradeoxy*galacto*sucrose tetra-acetate (2.2g) in methanol (20 ml) and acetone (0.5 ml) was treated with sodium methoxide to pH 9. The reaction was stirred at room temperature for 4 h, and tlc (dichloromethane—methanol 6:1) revealed a single product. The solution was neutralized by addition of Amberlyst 15 ($H^+$) resin, filtered and concentrated to dryness. The yield was 1.6g from tetra-acetate intermediate. The deacetylated compound was identical with that obtained in Example 3, Route 2, at (f).

Similarly, 4,1′,4′,6′-tetrachloro-4,1′,4′,6′-tetradeoxy*galactosorbo*sucrose was prepared from the corresponding tetra-acetate $[\alpha]_D$ + 71.3° [*c* 1.3, $H_2O$).

| | | | | |
|---|---|---|---|---|
| Anal: calc; | $C_{12}H_{18}O_7Cl_4$ | C 34.61, | H 4.32, | Cl 34.13% |
| found | | 35.5 | 4.84 | 34.2 |

The $^{13}C$ nmr of this *galactosorbo* product is included in the table given in Example 3.

Example 2

Chlorination of TGS 6-0-t-butyldiphenylsilyl ether

To TGS (2.0g) in pyridine (25 ml) was added dimethylaminopyridine (0.5g) followed by t-butyldiphenylsilyl chloride (1.4 ml, approx. 1.1 eq) and the mixture was heated with stirring at 50 to 60° for 3 days. The reaction mixture was then poured into ice-water and the syrupy product was washed by decantation with ice-water. It was then dissolved in chloroform, dried (sodium sulphate), concentrated to a syrup (3.0g, 93.7%) and crystallized from ethanol, mp 124—125°, $[\alpha]_D$+34.6°(*c* 0.8 $CHCl_3$). The syrup (2.0g) was dissolved in a mixture of pyridine (5.0ml) and chloroform (5.0ml) and cooled to −40°. Sulphuryl chloride (3g) was added dropwise and the temperature was slowly allowed to rise to room temperature and then raised with stirring to 45°. The mixture was stirred at this temperature for about 10 hours, again fitted with a calcium chloride tube as in Example 1. The mixture was then worked up as in Example 1 and the acetylated mixture was eluted from silica with petrol/ethyl acetate (5:1) to give a tetrachloro tetraacetate (2.5g 62%) as a syrup and TGS pentaacetate (approximately 10%). Crystallization of the tetrachloro derivative gave a pure compound apparently similar in its chlorination pattern of substitution to the compound obtained from sucrose 6-acetate. The product was then deacetylated (sodium methoxide in methanol) and desilylated (tetra-n-butylammonium fluoride) in tetrahydrofuran at room temperature to yield the free tetrachloro derivative identical to that in Example 1. Deacetylation and desilylation of the mother liquors from the crystallization gave a mixture of two compounds very similar in $R_f$ value on TLC, apparently identical to the two compounds obtained in Example 1, namely 1,4,6-trichloro-1,4,6-trideoxy-β-D-fructofuranosyl 4-chloro-4-deoxy-α-D-galactopyranoside and 4-chloro-4-deoxy-α-D-galactopyranosyl-1,4,6-trichloro-1, 4,6-trideoxy-β-D-sorbofuranoside.

Example 3

4,1',4',6'-tetrachloro-4,1',4',6'-tetradeoxy*galacto*sucrose
(1,4,6-trichloro-1,4,6-trideoxy-β-D-fructofuranosyl 4-chloro-4-deoxy-α-D-galactopyranoside)

Route 1

A solution of TGS (10g) in dry toluene (250 ml) was treated with DEAD (12ml, 2.3 molar equiv) followed to TPP (19g 1.3 m.e.). The reaction was exothermic, tlc (ether/petrol 7:1) after 5 min showed 2 major products. The mixture was refluxed 2.5 h. and then cooled and diluted with methanol (50 ml), concentrated to syrup, and taken up in ether. Most of the TPP oxide present was removed by crystallisation and the crude material was chromatographed on a column of silica gel (150 g), eluting with ether-light petroleum (1:1), to yield the 3,6-anhydro-3',4'-*lyxo*epoxide derivative of TGS (i.e., 3,6-anhydro-4-chloro-4-deoxy-α-D-galactopyranosyl 3,4-anhydro-1,6-dichloro-1,6-dideoxy-β-D-tagatofuranoside) (5g, 55%) $[\alpha]_D$ + 6.5° (C,1.0, $CHCl_3$)

| | | | |
|---|---|---|---|
| Anal: calc for $C_{12}H_{15}O_6Cl_3$ | C 39.83, | H 4.14, | Cl 29.46% |
| | C 40.28 | H 4.28 | Cl 26.45% |

Further elution of the column gave TGS 3',4'-*lyxo*epoxide. This material was peracetylated by treatment with acetic anhydride, to give TGS 3',4'-*lyxo*epoxide triacetate (i.e. 4-chloro-4-deoxy-2,3,6-trio-*O*-acetyl-α-D-galactopyranosyl 3,4-anhydro-1,6-dichloro-1,6-dideoxy-β-D-tagatofuranoside), structure supported by 'Hnmr and m.s. (see below).

Route 2

(a) TGS 6-t-butyldiphenylsilyl ether

A solution of TGS (8g) in dry pyridine was treated with *t*-BDPS chloride (5.6ml) and 4-dimethylamino-pyridine (200mg) at room temperature for 18 h. Tlc showed the presence of one major product together with some unreacted starting material (Tlc eluant:ethyl acetate/acetone/water, 10:10:1). The mixture was then poured into ice-water and extracted with ethyl acetate. The extracts were dried ($Na_2SO_4$) and evaporated to dryness. Crystallization from ethanol gave TGS *t*-BDPS ether (10.5g, 82.6%) m.p. 95—97° (toluene-petrol), $[\alpha]_D$+39.3° (c 1.0 $CHCl_3$)

| | | | |
|---|---|---|---|
| Anal: calc $C_{28}H_{37}O_8Cl_3Si$ | C 52.87, | H 5.82, | Cl 16.75% |
| found | 52.28 | 5.76% | |

(b) TGS 6-t-BDPS ether 3',4'-*lyxo*epoxide

A solution of TGS-*t*-BDPS ether (10g) in dry toluene (250 ml) was treated with DEAD (12 ml, 2.3 m.e.) followed by TPP (19g, 1.3 m.e.). The reaction was exothermic, tlc after 5 min (ether/acetone, 10:1) showed one major product and the absence of starting material. The reaction mixture was diluted with methanol (50 ml) concentrated to syrup and taken up in ether. Most of the TPP oxide by product was removed by crystallization and the crude material was chromatographed on a column of dry silica gel (150 g) with ether/light petroleum (2:1), then gradually increasing polarity to 4:1 and finally with ether/acetone (9:1), to yield the epoxide (8.5g, 87.6%).

(c) Peracetylation

Conventional acetylation of the product of stage (b) (7g) using pyridine (70 ml) and acetic anhydride (7 ml) gave the diacetate (7.5g, 94.8%) $[\alpha]_D$ + 104.5° (c 1.0, $CHCl_3$)

| | | | |
|---|---|---|---|
| Anal: calc for $C_{32}H_{39}O_9Cl_3Si$ | C 54.73, | H 5.55, | Cl 15.18% |
| found | 55.42 | 5.76 | |

(d) TGS 3',4'-*lyxo*epoxide triacetate

A solution of the diacetate from stage (c) (7g) in tetrahydrofuran (150 ml) was treated with tetrahydrofuran (150 ml) was treated with tetra-*n*-butylammonium fluoride (1.4 g) at room temperature for 18 h. Tlc (ether/light petroleum, 6:1) showed one major product with traces of slow moving products due to partial deacetylation. The mixture was concentrated, taken up in dry pyridine (50 ml) and treated with acetic anhydride (7 ml) at room temperature for 3 h. Tlc ether/light petroleum (7:1) showed only one product. The reaction mixture was concentrated and was eluted from a short column of silica gel (50 g) with ether/light petroleum (1:1) to give a crystalline product (4.3 g, 85.2%) identical with the product from Route 1, m.p. 133—134° $[\alpha]_D^{20}$ + 116.3° (c 1.0, $CHCl_3$)

| | | | |
|---|---|---|---|
| Anal: calc for $C_{18}H_{23}O_{10}Cl_3$ | C 42.72, | H 5.54, | Cl 21.06% |
| found | 43.00 | 4.58 | 20.79 |

(e) 4,1',4',6'-tetrachloro-4,1',4',6'-tetradeoxy*galacto*sucrose tetraacetate

A solution of the product from stage (d) in dmf (50 ml) was treated with lithium chloride (4g) at

90° for 5 h. The reaction mixture was poured into ice-water and extracted with ether. The extracts were dried ($Na_2SO_4$), concentrated to a syrup and acetylated in the normal manner with pyridine and acetic anhydride to yield the tetrachloro tetraacetate (2.6g, 56.2%) m.p. 103—104° (ether/light petroleum) $[\alpha]_D$ + 75.0 (c,1.0,$CHCl_3$)

Anal: calc $C_{20}H_{26}O_{11}Cl_4$ C 41.09, H 4.45%
found 41.43 4.53%

(f) 4,1',4',6'-tetrachloro-4,1',4',6'-tetradeoxy*galacto*sucrose

A solution of the tetraacetate from stage (e) (1.5g) in dry methanol (25ml) was treated with a catalytic amount of sodium methoxide at room temperature for 5h, deionized by being stirred with Amberlyst 15 resin (T.M.) and concentrated to dryness. Crystallization from ether gave the product (1g,93.5%) m.p. 58—60 $[\alpha]_D^{20}$ + 72.3° (c 1.0,$H_2O$)

Anal: calc for $C_{12}H_{18}O_7Cl_4$ C 34.61, H 4.32, Cl 34.13%
found 35.5 4.84 34.2%
Structure consistent with $^{13}C$ nmr spectrum

$^{13}C$ N.m.r. Chemical Shifts[a]

| | Sucrose[b] | TGS | 4,1',4',6' tetrachloro 4,1',4'6'tetradeoxy galactosucrose | 4,1',4',6' tetrachloro 4,1',4'6'tetradeoxy galactosorbosucrose[c] |
|---|---|---|---|---|
| C—2' | 104.4 | 104.1 | 103.7 | 105.1 |
| C—1 | 92.9 | 93.5 | 93.1 | 94.7 |
| C—5' | 82.2 | 81.9 | 82.5 | 80.9 |
| C—3' | 77.4 | 76.9 | 77.7 | 74.2 |
| C—4' | 74.8 | 76.1 | 59.4 | 61.2 |
| C—5 | 73.2 | 71.4 | 71.0 | 71.3 |
| C—3 } | 73.5 | 68.8 / | 68.5 / | 69.1 / |
| C—2 } | 71.9 | /68.4 | /68.1 | /68.9 |
| C—4 | 70.1 | 63.9 | 63.4 | 64.1 |
| C—1' | 63.3 | 45.6 | 44.3 | 44.3 |
| C—6' | 62.5 | 44.4 | 44.0 | 44.0 |
| C—6 | 61.2 | 62.2 | 61.8 | 62.7 |

[a]Chemical Shifts are expressed in p.p.m. downfield from the $^{13}C$ N.m.r. resonance of tetramethylsilane. $D_2O$ was used as solvent and D.S.S. as internal standard.
[b]Data from Development in Food Carbohydrates-2 C.K. Lee.
[c]From Example 1.

$^1$Hnmr parameters. First order chemical shifts (δ) and coupling constants (Hz) at 220 MHz.

| | TGS 3′4′ epoxide triacetate | TGS 3′4′ epoxide 6t-BDPS ether diacetate | 4,1′,4′,6′ tetrachloro-4,1′,4′,6′-tetradeoxy *galacto* sucrose |
|---|---|---|---|
| H—1 | 5.82d $J_{1,2}$ 3.2 | 5.80d $J_{1,2}$ 3.24 | 5.72d $J_{1,2}$ 3.5 |
| H—2 | 5.13dd $J_{2,3}$ 8.52 | 5.13dd $J_{2,3}$ 8.52 | 5.28dd $J_{2,3}$ 3.0 |
| H—3 | 5.31dd $J_{3,4}$ 2.94 | 5.35dd $J_{3,4}$ 2.94 | 5.28dd $J_{3,4}$ 2.0 |
| H—4 | 4.60t $J_{4,5}$ 1.18 | 4.71q $J_{4,5}$ 1.18 | 4.76d $J_{4,5}$ 2.0 |
| H—5 | 4.55m | 4.14m | 4.55m |
| H—6 | | | |
| H—3′ | 3.92d $J_{3',4'}$ 2.04 | 3.82d $J_{3',4'}$ 2.06 | 5.65d $J_{3',4'}$ 9.0 |
| H—4 | 3.90q $J_{4',5'}$ 2.22 | 3.79q $J_{4',5'}$ 2.52 | 4.37d $J_{4'5'}$ 2.0 |
| H—5′ | 4.18m | | 4.32m |
| H—6′ | | | |
| H—1′a | 4.28d $J_{1'a,1'b}$ 5.30 | | |
| H—1′b | 4.24d | | |

Mass spectroscopic analysis

a = 3:1 doublets 1 Cl
b = 27:27:9:1 quartet 3 Cl
c = 9:6:1 triplet 2 Cl

| Compound No. | Hexopyranosyl cation m/e | Ketofuranosyl cation |
|---|---|---|
| 7<br>TGS 6-*t*-BDPS<br>$Ac_2$<br>3′4′ epoxide | 503<br>247 (a)<br>↙ ↘<br>187 205<br>↘ ↙<br>145 (a)<br>109 | 181 (c)<br>165 (c)<br>129 (a) |
| 4<br>TGS<br>$(Ac)_3$<br>3′4′ epoxide | 307 (a)<br>247 (a)<br>187 (a)<br>145 (a)<br>109 | 181 (c)<br>165 (c)<br>129 (a) |
| 8<br>$(Cl)_4\,(OAc)_4$ | 307 (a)<br>247 (a)<br>187 (a)<br>145 (a)<br>109 | 259 (a)<br>223 (a)<br>181 (c)<br>145 (a) |

X Ray Crystallographic Data

X-ray crystallographic of a crystalline sample of 4,1′,4′,6′-tetrachloro-4,1,4′,6′-tetradeoxy *galacto*sucrose tetraacetate gave the following coordinates:

| ATOM (position) | ATOMIC CO-ORDINATES ($X10^4$) WITH ESTIMATED STANDARD DEVIATIONS IN PARENTHESES | | |
|---|---|---|---|
| | X | Y | Z |
| CL(4) | 6370(1) | 2145(2) | 5341(1) |
| C(1) | 3987(3) | 2186(5) | 6598(4) |
| C(2) | 5127(3) | 2220(5) | 7481(4) |
| O(2) | 5213(3) | 2347(4) | 8806(3) |
| C(2,1) | 4887(4) | 1197(5) | 9340(5) |
| O(2,1) | 4454(3) | 180(4) | 8724(4) |
| C(2,2) | 5119(6) | 1411(9) | 10757(6) |

| | X | Y | Z |
|---|---|---|---|
| C(3) | 5751(3) | 3456(5) | 7198(5) |
| O(3) | 6816(3) | 3281(4) | 7987(4) |
| C(3,1) | 7399(4) | 4473(6) | 8417(5) |
| O(3,1) | 7080(4) | 5641(5) | 8092(5) |
| C(3,2) | 8488(5) | 4069(9) | 9279(7) |
| C(4) | 5635(4) | 3542(5) | 5780(5) |
| O(5) | 3983(2) | 2220(3) | 5314(3) |
| C(5) | 4472(4) | 3464(5) | 4995(4) |
| C(6) | 4255(4) | 3441(5) | 3568(5) |
| O(6) | 3172(3) | 3905(5) | 3003(3) |
| C(6,1) | 2851(5) | 4174(7) | 1755(5) |
| O(6,1) | 3416(4) | 4052(8) | 1106(4) |
| C(6,2) | 1733(6) | 4666(13) | 1266(6) |
| O(1) | 3436(2) | 3373(3) | 6871(3) |
| CL(1′) | 1143(1) | 2462(2) | 8333(1) |
| CL(4′) | 387(1) | 5056(2) | 3799(1) |
| CL(6′) | 1271(1) | −252(2) | 3908(1) |
| C(1′) | 2409(4) | 2776(6) | 8190(5) |
| O(2′) | 1902(2) | 1956(3) | 6053(3) |
| C(2′) | 2358(3) | 3120(5) | 6845(4) |
| C(3′) | 1701(4) | 4408(5) | 6218(4) |
| O(3′) | 2225(3) | 5765(4) | 6499(4) |
| C(3′,1) | 1908(4) | 6694(6) | 7249(5) |
| O(3′,1) | 1309(5) | 6350(7) | 7777(6) |
| C(3′,2) | 2397(6) | 8139(7) | 7282(7) |
| C(4′) | 1450(4) | 4042(5) | 4819(4) |
| C(5′) | 1205(3) | 2454(5) | 4820(4) |
| C(6′) | 1415(4) | 1642(6) | 3749(5) |

The dimensions of the unit cell are:—
a = 13.99 Å
b = 9.37 Å
c = 10.99 Å
= 108.7° Monoclinic; space group $P2_1$

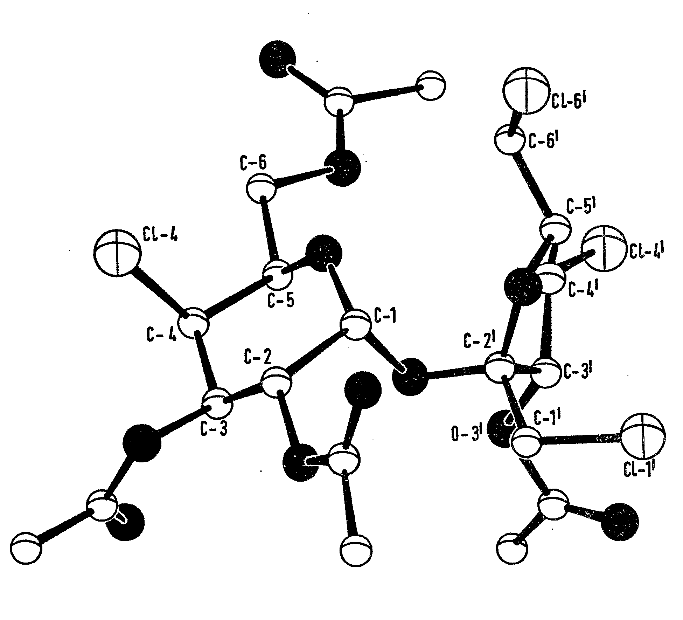

A computer-generated view of the molecule is shown in the accompanying figure, which clearly shows that the configuration at positions 3′ and 4′ is the *fructo* configuration.

Example 4

Sweetening tablets for beverages

Each tablet contains 1,4,6-trichloro-1,4,6-trideoxy-β-D-fructofuranosyl 4-chloro-4-deoxy-α-galactopyranoside from Example 1, Example 2 or Example 3 (2 mg) together with dispersible tablet base (circa 60 mg) containing sucrose, gum arabic and magnesium stearate.

Example 5

Reduced calorie cola drink containing sugar

Ingredients to prepare 100 ml bottling syrup:

| | |
|---|---|
| tetrachloro derivative of Example 3 | 20 mg |
| sucrose | 60 g |
| benzoic acid | 35 mg |
| phosphoric acid | (conc) 1 ml |
| cola flavour | 1.1 ml |
| colour | ad. lib |
| mineral water | ad.100 ml |

This syrup may then be added in 20 ml doses to carbonated 225 ml aliquots of chilled mineral water.

Example 6

Toothpaste

| | % by weight |
|---|---|
| Dibasic calcium phosphate | 50% |
| Glycerol | 20% |
| Sodium lauryl sulphate | 2.5% |
| Spearmint oil | 2.5% |
| Gum tragacanth | 1.0 % |
| Tetrachloro derivative of Example 3 | 0.03% |
| Water | 23.97% |

The ingredients are mixed to produce a spearmiunt flavoured toothpaste of acceptable sweetness for free from sugar or saccharin.

Example 7

Chewing gum

| | part by weight |
|---|---|
| Polyvinyl acetate | 20 |
| Butyl phthalybutylglycolate | 3 |
| Polyisobutylene | 3 |
| Microcrystalline wax | 2 |
| Calcium carbonate | 2 |
| Flavouring/aroma | 1 |
| Tetrachloro derivative of Example 3 | 0.07 |
| Glucose | 10 |

The above chewing gum base can be cut into conventional tablets or strips.

**Claims for the Contracting States: BE CH DE FR IT LI LU NL SE**

1. A compound of the general formula

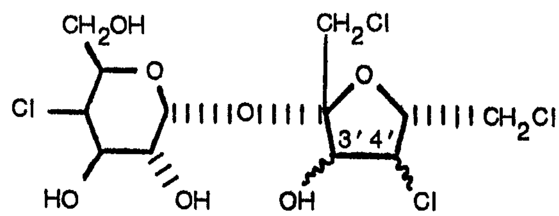

I

(in which the 3′ and 4′- substituents have a trans-configuration relative to each other).

2. 1,4,6-Trichloro-1,4,6-trideoxy-β-D-fructofuranosyl 4-chloro-4-deoxy-α-D-galactopyranoside.

3. 4-Chloro-4-doexy-α-D-galactopyranosyl 1,4,6-trichloro- 1,4,6-trideoxy-β-D-sorbofuranoside.

4. A process for the preparation of a compound as defined in claim 1, comprising direct chlorination of a sucrose derivative blocked in the 6- position but possessing free hydroxy groups in the 4, 1′, 4′ and 6′- positions, and subsequent removal of the blocking group from the 6- position.

5. A process according to claim 4, in which the chlorination is effected using sulphuryl chloride.

6. A process for the preparation of 1,4,6-trichloro-1,4,6-trideoxy-β-D-fructofuranosyl 4-chloro-4-deoxy-α-D-galactopyranoside comprising chlorination of 4-chloro-4-deoxy-α-D-fructofuranosyl β-D-glucopyranoside blocked at the 6- position, and subsequent removal of the blocking group.

7. A process according to claim 6, in which the chlorination is effected using sulphuryl chloride or a Vilsmeier reagent.

8. A process according to any of claims 4 to 7, in which the blocking group is an ester group.

9. An ingestible product or oral composition containing at least one compound according to claim 1 as a sweetening agent.

10. A sweetening composition containing at least one compound according to claim 1 as a sweetening agent.

11. A method of sweetening a substance comprising incorporating therein at least one compound according to claim 1 as a sweetening agent.

**Claims for the Contracting State: AT**

1. A process for the preparation of a compound of the general formula

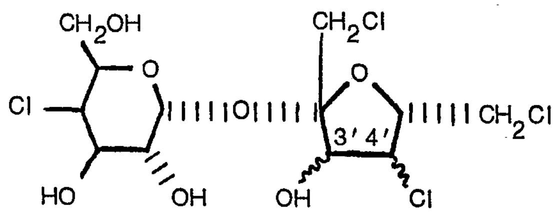

I

(in which the 3′ and 4′- substituents have a trans-configuration relative to each other comprising direct chlorination of a sucrose derivative blocked in the 6- position but possessing free hydroxy groups in the 4, 1′, 4′ and 6′- positions, and subsequent removal of the blocking group from the 6- position).

2. A process according to claim 1, in which the chlorination is effected using sulphuryl chloride.

3. A process for the preparation of 1,4,6-trichloro- 1,4,6-trideoxy-β-D-fructofuranosyl 4-chloro-4-deoxy-α-D-galactopyranoside comprising chlorination of 4-chloro-4-deoxy-β-D-fructofuranosyl α-D-glucopyranoside blocked at the 6- position, and subsequent removal of the blocking group.

4. A process according to claim 3, in which the chlorination is directed by sulphuryl chloride or a Vilsmeier reagent.

5. A process according to any of claims 1 to 4, in which the blocking group is an ester group.

6. A compound of the general formula I

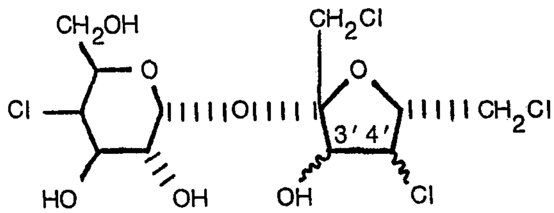

(in which the 3′ and 4′- substituents have a trans-configuration relative to each other when prepared by a process as claimed in any of claims 1 to 5 or an obvious chemical equivalent thereof.

7. 1,4,6-Trichloro-1,4,6-trideoxy-β-D-fructofuranosyl 4-chloro-4-deoxy-α-D-galactopyranoside when prepared by a process as claimed in any of claims 1 to 5 or an obvious chemical equivalent thereof.

8. 4-Chloro-4-deoxy-α-D-galactopyranosyl 1,4,6-trichloro-1, 4,6-trideoxy-3-D-sorbofuranoside when prepared by a process as claimed in claim 1 or an obvious chemical equivalent thereof.

9. A process of producing an ingestible product or oral composition by incorporating therein at least one compound of formula I

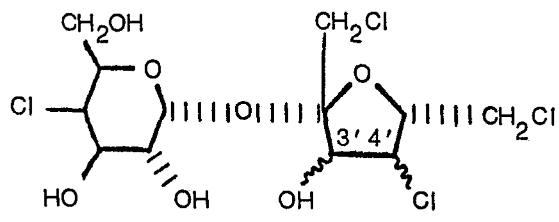

(in which the 3′ and 4′- substituents have a trans configuration relative to each other) as a sweetening agent.

10. A process of producing a sweetening composition comprising production of at least one compound of formula I by a process according to any of claims 1 to 5 and formulating it together with an extender or carrier.

11. A method of sweetening a substance comprising incorporating therein at least one compound according to claim 6 as a sweetening agent.

**Revendications pour les Etats contractants: BE CH DE FR IT LI LU NL SE**

1. Composé de formule générale

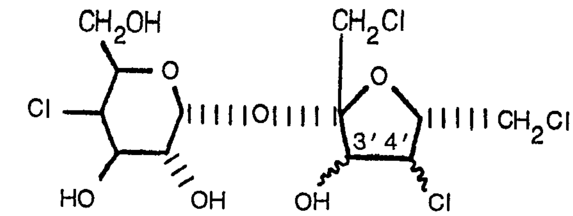

I

(dans laquelle les substituants en 3′ et 4′ ont une disposition trans l'un par rapport à l'autre).

2. 1,4,6-trichloro-1,4,6-tridésoxy-β-D-fructofurannosyl 4-chloro-4-désoxy-α-D-galactopyrannoside.

3. 4-chloro-4-désoxy-α-D-galactopyrannosyl 1,4,6-trichloro-1,4,6-tridésoxy-β-D-sorbofurannoside.

4. Procédé pour la préparation d'un composé selon la revendication 1, caractérisé en ce qu'il comprend la chloration directe d'un dérivé du saccharose bloqué en position 6, mais possédant des groupes hydroxy libres en positions 4, 1′, 4′ et 6′, puis la suppression du groupe de blocage de la position 6.

5. Procédé selon la revendication 4, caractérisé en ce que la chloration est effectuée au moyen de chlorure de sulfuryle.

6. Procédé pour la préparation de 1,4,6-trichloro-1,4,6-tridésoxy-β-D-fructofurannosyl 4-chloro-4-désoxy-α-D-galactopyrannoside, caractérisé en ce qu'il comprend la chloration de 4-chloro-4-désoxy-α-D-fructofurannosyl β-D-glucopyrannoside bloqué en position 6, puis la suppression du groupe de blocage.

7. Procédé selon la revendication 6, caractérisé en ce que la chloration est effectuée au moyen de chlorure de sulfuryle ou d'un réactif de Vilsmeier.

8. Procédé selon l'une quelconque des revendications 4 à 7, caractérisé en ce que le groupe de blocage est un groupe ester.

9. Produit ingérable ou composition orale, contenant au moins un composé selon la revendication 1 en tant qu'agent édulcorant.

10. Composition édulcorante, contenant au moins un composé selon la revendication 1 en tant qu'agent édulcorant.

11. Procédé pour sucrer une substance, consistant à y incorporer au moins un composé selon la revendication 1 en tant qu'agent édulcorant.

**Revendications pour l'Etat contractant: AT**

1. Procédé pour la préparation d'un composé de formule générale

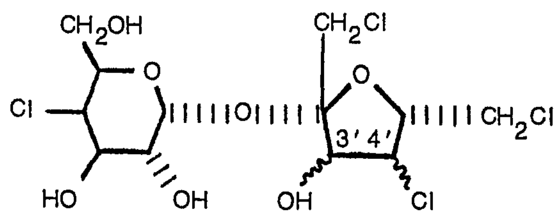

I

(dans laquelle les substituants en 3′ et 4′ ont une disposition trans l'un par rapport à l'autre), comprenant une chloration directe d'un dérivé du saccharose bloqué en position 6, mais possédant des groupes hydroxy libres dans les positions 4, 1′, 4′ et 6′, puis la suppression du groupe de blocage de la position 6.

2. Procédé selon la revendication 1, caractérisé en ce que la chloration est effectuée au moyen de chlorure de sulfuryle.

3. Procédé pour la préparation de 1,4,6-trichloro-1,4,6-tridésoxy-β-D-fructofurannosyl 4-chloro-4-désoxy-α-D-galactopyrannoside, caractérisé en ce qu'il comprend la chloration de 4-chloro-4-désoxy-β-D-fructofurannosyl α-D-glucopyrannoside bloqué en position 6, puis la suppression du groupe de blocage.

4. Procédé selon la revendication 3, caractérisé en ce que la chloration est assurée par le chlorure de sulfuryle ou un réactif de Vilsmeier.

5. Procédé selon l'une quelconque des revendications 1 à 4, caractérisé en ce que le groupe de blocage est un groupe ester.

6. Composé de formule générale I

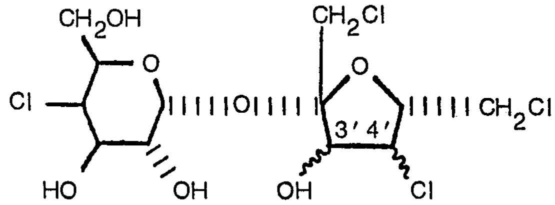

(dans laquelle les substituants en 3′ et 4′ ont une disposition trans l'un par rapport à l'autre, lorsqu'il est préparé par un procédé selon l'une quelconque des revendications 1 à 5 ou équivalent chimique évident d'un tel composé.

7. 1,4,6-trichloro-1,4,6-tridésoxy-β-D-fructofurannosyl 4-chloro-4-désoxy-α-D-galactopyrannoside, lorsqu'il est préparé par un procédé selon l'une quelconque des revendications 1 à 5, ou équivalent chimique évident de ce composé.

8. 4-chloro-4-désoxy-α-D-galactopyrannosyl 1,4,6-trichloro-1,4,6-tridésoxy-β-D-sorbofurannoside, lorsqu'il est préparé par un procédé selon la revendication 1, ou équivalent chimique évident de ce composé.

9. Procédé de fabrication d'un produit ingérable ou d'une composition orale, caractérisé en ce qu'il consiste à y incorporer au moins un composé de formule I

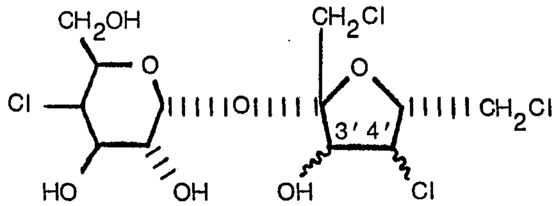

(dans laquelle les substituants en 3′ et 4′ ont une disposition trans l'un par rapport à l'autre) en tant qu'agent édulcorant.

10. Procédé de fabrication d'une composition édulcorante, caractérisé en ce qu'il consiste à préparer au moins un composé de formule I par un procédé selon l'une quelconque des revendications 1 à 5 et à le formuler avec un diluant ou un excipient.

11. Procédé d'édulcoration d'une substance, caractérisé en ce qu'il consiste à y incorporer au moins un composé selon la revendication 6 en tant qu'agent édulcorant.

**Patentansprüche für die Vertragsstaaten: BE CH DE FR IT LI LU NL SE**

1. Verbindung der allgemeinen Formel

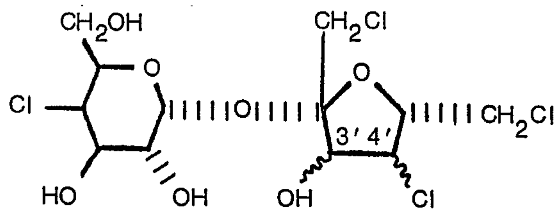

I

(worin die 3′- und 4′-Substituenten zueinander in trans-Konfiguration stehen).

2. 1,4,6-Trichlor-1,4,6-trideoxy-β-D-fructofuranosyl-4-chlor-4-deoxy-α-D-galactopyranosid.

3. 4-Chlor-4-deoxy-α-D-galactopyranosyl-1,4,6-trichlor-1,4,6-trideoxy-β-D-sorbofuranosid.

4. Verfahren zum Herstellen einer Verbindung gemäß Anspruch 1, bei welchem ein in 6-Stellung blockiertes, jedoch in 4-, 1′-, 4′- und 6′-Stellung freie Hydroxygruppen aufweisendes Saccharosederivat direkt chloriert und sodann die Blockiergruppe aus der 6-Stellung entfernt wird.

5. Verfahren nach Anspruch 4, bei welchem die Chlorierung unter Verwendung von Sulfurylchlorid durchgeführt wird.

6. Verfahren zum Herstellen des 1,4,6-Trichlor-1,4,6-trideoxy-β-D-fructofuranosyl-4-chlor-4-deoxy-α-D-galactopyranosids, bei welchem in 6-Stellung blockiertes 4-Chlor-4-deoxy-α-D-fructofuranosyl-β-D-glucopyranosid chloriert und anschließend die Blockiergruppe abgespalten wird.

7. Verfahren nach Anspruch 6, bei welchem das Chlorieren unter Verwendung von Sulfurylchlorid oder eines Vilsmeier-Reagens durchgeführt wird.

8. Verfahren nach irgendeinem der Ansprüche 4 bis 7, bei welchem die Blockiergruppe eine Estergruppe ist.

9. Einnehmbares Produkt oder orale Mischung, welche zumindest eine Verbindung gemäß Anspruch 1 als Süßstoff enthält.

10. Süßungsmittel, welche zumindest eine Verbindung gemäß Anspruch 1 als einen Süßstoff enthält.

11. Verfahren zum Süßen einer Substanz, bei welchem darin zumindest eine Verbindung gemäß Anspruch 1 als Süßstoff eingebracht wird.

**Patentansprüche für den Vertragsstaat: AT**

1. Verfahren zum Herstellen einer Verbindung der allgemeinen Formel

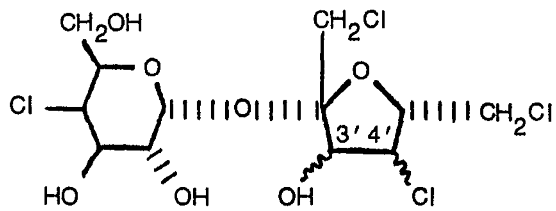

I

(worin die 3'- und 4'-Substituenten zueinander trans-Konfiguration besitzen), bei welchem ein in 6-Stellung blockiertes, jedoch in 4-, 1'-, 4'- und 6'-Stellung freie Hydroxygruppen aufweisendes Saccharosederivat direkt chloriert und anschließend die Blockiergruppe aus der 6-Stellung entfernt wird.

2. Verfahren nach Anspruch 1, bei welchem das Chlorieren unter Verwendung von Sulfurylchlorid, durchgeführt wird.

3. Verfahren zum Herstellen des 1,4,6-Trichlor-1,4,6-trideoxy-$\beta$-D-fructofuranosyl-4-chlor-4-deoxy-$\alpha$-D-galactopyranosids, bei welchem in 6-Stellung blockiertes 4-chlor-4-deoxy-$\beta$-D-fructofuranosyl-$\alpha$-D-glucopyranosid chloriert und anschließend die Blockiergruppe entfernt wird.

4. Verfahren nach Anspruch 3, bei welchem das Chlorieren mit Sulfurylchlorid oder einem Vilsmeier-Reagens vorgenommen wird.

5. Verfahren nach irgendeinem der Ansprüche 1 bis 4, bei welchem die Blockiergruppe eine Estergruppe ist.

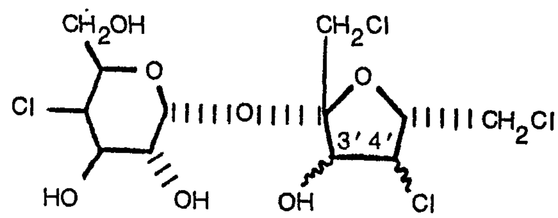

(in welcher die 3'- und 4'-Substituenten zueinander in trans-Konfiguration stehen), welche gemäß irgendeinem der Ansprüche 1 bis 5 oder einem offensichtlichen chemischen Äquivalent hievon hergestellt worden ist.

7. 1,4,6-Trichlor-1,4,6-trideoxy-$\beta$-D-fructofuranosyl-4-chlor-4-deoxy-$\alpha$-D-galactopyranosid, hergestellt nach einem Verfahren gemäß irgendeinem der Ansprüche 1 bis 5 oder einem offensichtlichen chemischen Äquivalent hievon.

8. 4-Chlor-4-deoxy-$\alpha$-D-galactopyranosyl-1,4,6-trichlor-1,4,6-trideoxy-$\beta$-D-sorbofuranosid, hergestellt nach einem Verfahren gemäß Anspruch 1 oder einem offensichtlichen chemischen Äquivalent hievon.

9. Verfahren zum Herstellen eines einnehmbaren Produktes oder einer oralen Mischung durch Einbringen zumindest einer Verbindung der Formel (I)

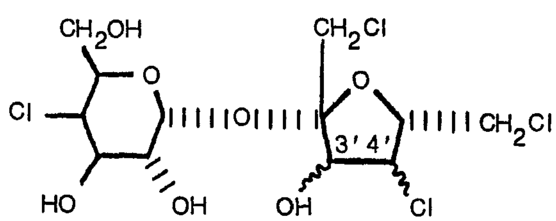

(in welcher die 3'- und 4'-Substituenten zueinander in trans-Konfiguration stehen) als Süßstoff in die Mischung.

10. Verfahren zum Herstellen eines Süßungsmittels, welches die Herstellung zumindest einer Verbindung der Formel (I) durch ein Verfahren gemäß irgendeinem der Ansprüche 1 bis 5 und Formulieren derselben zusammen mit einem Extender oder Träger umfaßt.

11. Verfahren zum Süßen einer Substanz, welches das Einbauen darin zumindest einer Verbindung gemäß Anspruch 6 als Süßstoff umfaßt.